# EUROPEAN PATENT APPLICATION

(11) **EP 2 548 514 A1**
(43) Date of publication of application: **23.01.2013**
(21) Application number: 11756020.1
(22) Date of filing: 17.02.2011
(51) Int. Cl.: A61B 8/08

(54) **ULTRASOUND DIAGNOSTIC DEVICE AND ULTRASOUND IMAGE DISPLAY METHOD**

(30) Priority: 19.03.2010 JP 2010063410
(71) Applicant: Hitachi Medical Corporation, Chiyoda-ku Tokyo 101-0021 (JP)
(72) Inventor: SAWAYAMA,Yuki, Tokyo 101-0021 (JP); WAKI,Koji, Tokyo 101-0021 (JP); IIMURA,Takashi, Tokyo 101-0021 (JP)
(74) Representative: MERH-IP Matias Erny Reichl Hoffmann
(86) International application number: PCT/JP2011/053320
(87) International publication number: WO 2011/114830

(57) **Abstract**

An ultrasonic diagnostic apparatus comprises an operation unit that arbitrarily specifies a cross-section; a specified cross-section tomographic image constructing unit that cuts and constructs a tomographic image of the specified cross-section from tomographic volume data that is stored in the volume data storing unit; a specified cross-section elastic image constructing unit that cuts and constructs an elastic image of the specified cross-section from elastic volume data that is stored in the volume data storing unit; and a display unit that displays in parallel the tomographic image and the elastic image that are constructed by the specified cross-section tomographic image constructing unit and the specified cross-section elastic image constructing unit.

## Description

### Field of the Invention

The present invention relates to an ultrasonic diagnostic apparatus and an ultrasonic image display method for displaying a tomographic image or an elastic image showing the hardness or softness of biological tissue of an object using ultrasonic waves.

### Description of Related Art

An ultrasonic diagnostic apparatus has been used for transmitting ultrasonic waves to the inside of an object to be examined via an ultrasonic probe, generating volume data by receiving the reflected echo signals in accordance with the configuration of the biological tissue from the inside of the object, and displaying a 3-dimensional tomographic image and a 3-dimensional elastic image (for example, Patent Document 1) . Further, a method is proposed to cut out an arbitrary cross-section from the obtained volume data and display a tomographic image (for example, Patent Document 2).

### Prior Art Documents

### Patent Documents

Patent Document 1: JP-A-2008-259605
Patent Document 2: U.S. Patent No. 6413219

The above-mentioned Patent Documents do not disclose the technique, with respect to the cross-section which is arbitrarily cut out from volume data, to juxtapose and display a tomographic image and an elastic image. Thus juxtaposing and displaying of an elastic image and a tomographic image of the cross-section which is arbitrarily cut out from volume data has not been possible to perform.

Given this factor, the objective of the present invention is to provide a technique for displaying in parallel an elastic image and a tomographic image of a cross-section which is arbitrarily cut out from volume data.

### Brief Summary of the Invention

In order to achieve the above-described objective, the ultrasonic diagnostic apparatus of the present invention comprises:
an ultrasonic probe provided with transducers that transmit/receive ultrasonic waves;
a transmission unit configured to transmit ultrasonic waves to an object via an ultrasonic probe;
a reception unit configured to receive the reflected echo signals from an object;
a volume data storing unit configured to store tomographic volume data and elastic volume data created by processing the reflected echo signals;
an operation unit configured to arbitrarily specify a cross-section;
a specified cross-section tomographic image constructing unit configured to cut out and construct a tomographic image of the specified cross-section from the tomographic volume data stored in the volume data storing unit;
a specified cross-section elastic image constructing unit configured to cut out and construct an elastic image of the specified cross-section from the elastic volume data stored in the volume data storing unit; and
a display unit configured to juxtapose and display the tomographic image and the elastic image constructed by the specified cross-section tomographic image constructing unit and the specified cross-section elastic image constructing unit. In addition, the tomographic image and the elastic image here are cut out from the same specified cross-section.

Also, the ultrasonic diagnostic apparatus of the present invention further comprises a synthesis processing unit configured to synthesize a tomographic image and an elastic image to create a synthetic image, and the display unit displays the tomographic image and the synthetic image in parallel.

### Effect of the Invention

In accordance with the present invention, it is possible to juxtapose and display an elastic image and a tomographic image with respect to a cross-section which is arbitrarily cut out from volume data.

### Brief Description of the Drawings

Fig. 1 shows the configuration of the ultrasonic diagnostic apparatus related to the present invention.
Fig. 2 is a display pattern of a specified cross-section in an image display unit 109 of the present invention.
Fig. 3 is an MPR display pattern in the image display unit 109 of the present invention.
Fig. 4 is a display pattern of plural cross-sections that are parallel to each other in the image display unit 109 of the present invention.
Fig. 5 shows third and fourth embodiments of the present invention.
Fig. 6 shows the fourth embodiment of the present invention.
Fig. 7 shows a fifth embodiment of the present invention.

### Detailed Description of the Invention

The present invention will be described referring to the attached diagrams.

### Embodiment 1

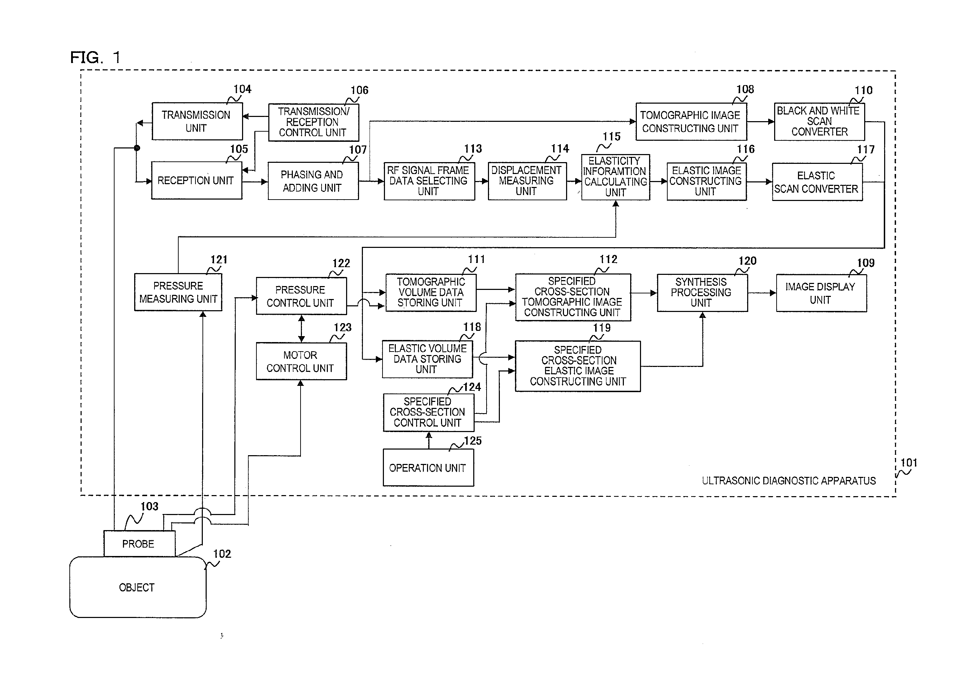

Fig. 1 shows the configuration of an ultrasonic diagnostic apparatus 101 related to the present invention. As shown in Fig. 1, the ultrasonic diagnostic apparatus comprises an ultrasonic probe 103 to be used by applying to an object 102, a transmission unit 104 configured to repeatedly transmit ultrasonic waves to the object 102 via the ultrasonic probe 103 at intervals, a reception unit 105 configured to receive the time-series reflected echo signals generated from the object 102, a transmission/reception control unit 106 configured to control the transmission unit 104 and the reception unit 105, and a phasing and adding unit 107 configured to perform phasing and adding of the reflected echo signals received by the reception unit 105.

The ultrasonic probe 103 is provided with a plurality of transducers arrayed therein, to transmit/receive ultrasonic waves to/from the object 102 via the transducers. The ultrasonic probe 103 is formed by a plurality of rectangle or fan-shaped transducers, and is capable of mechanically vibrating the transducers in the direction orthogonal to the array direction of the plural transducers and transmitting/receiving ultrasonic waves 3-dimensionally.

Also, the ultrasonic probe 102 may also be provided with 2-dimensional array of plural transducers to electronically control the transmission/reception of ultrasonic waves. In concrete terms, the transducers arrayed on the ultrasonic transmitting/receiving surface of the ultrasonic probe 103 are cut off plurally also in the minor axis direction, and are 2-dimensionally arrayed for 1~k channels. Therefore, the ultrasonic probe 103 is capable of scanning the ultrasonic beams generated by electronic focusing in the minor axis direction along the curvature of the ultrasonic transmitting/receiving surface or the minor axis direction, so as to 3-dimensionally collect the RF signal frame data. In addition, the ultrasonic probe 103 can execute measurement in the transmitting/receiving direction (*θ*,*φ*) at the same time as transmitting/receiving ultrasonic waves.

The transmission unit 104 generates transmission pulses for generating ultrasonic waves by activating the transducers of the ultrasonic probe 103. The transmission unit 104 has a function to set the convergent point of the transmitted ultrasonic waves at a certain depth. Also, the reception unit 105 amplifies the reflected echo signal received by the ultrasonic probe 103 at a predetermined gain, and generates an RF signal, i.e. a reception signal. The transmission/reception control unit 106 controls devices such as the transmission unit 104 and the reception unit 106.

The phasing and adding unit 107 controls the phase of the RF signal amplified in the reception unit 105, and generates RF signal frame data (which is equivalent to RAW data) by forming an ultrasonic beam with respect to one or plural convergent points.

The ultrasonic diagnostic apparatus 101 is provided with a tomographic image constructing unit 108 configured to construct a tomographic image, for example a black and white tomographic image on the basis of the RF signal frame data which is generated by phasing and adding of the signals in the phasing and adding unit 107, a black and white scan converter 110 configured to convert the output signals from the tomographic image constructing unit 108 to match them for the display of the image display unit 109, a tomographic volume data storing unit 111 configured to store the tomographic image frame data output from the black and white scan converter 110 as the tomographic volume data, and a specified cross-section tomographic image constructing unit 112 configured to construct an arbitrary tomographic image from the tomographic volume data stored in the tomographic volume data storing unit 111.

The tomographic image constructing unit 108 inputs the RF signal frame data from the phasing and adding unit 107, and executes signal processing such as gain compensation, log compression, detection, edge enhancement or filtering so as to obtain tomographic image frame data. Also, the black and white scan converter 110 is configured including a coordinate converter, a frame memory for storing the converted plural pieces of tomographic image frame data in time series, and a controller. The black and white scan converter 110 obtains the tomographic image frame data in the object 102 stored in the frame memory as one image, reads out the obtained tomographic image frame data in synchronization with TV, and executes coordinate conversion on the tomographic image frame data to match the data with the display of the image display 109.

The plural sets of tomographic image frame data output from the black and white scan converter 110 are stored in the tomographic volume data storing unit 111. The tomographic volume data storing unit 111 executes 3-dimensional conversion with respect to the plural sets of tomographic image frame data on the basis of the transmitting/receiving direction (*θ*,*φ*) which is equivalent to the acquisition position of the tomographic image, generates and stores the tomographic volume data. The tomographic volume data in the tomographic volume data storing unit 111 is read out to the specified cross-section tomographic image constructing unit 112, and a tomographic image is constructed with respect to the cross-section which is arbitrarily specified.

The ultrasonic diagnostic apparatus 101 is also provided with an RF signal frame data selecting unit 113 configured to store the RF signal frame data output from the phasing and adding unit 107 and select at least two pieces of RF signal frame data, a displacement measuring unit 114 configured to measure the displacement in the biological tissue of the object 102, an elasticity information calculating unit 115 configured to acquire the strain or the elasticity modulus from the displacement information measured in the displacement measuring unit 114, an elastic image constructing unit 116 configured to construct a color elastic image from the strain or the elasticity modulus calculated in the elasticity information calculating unit 115, an elastic scan converter 117 configured to convert the output signals from the elastic image constructing unit 116 for matching the signals to the display of the image display unit 109, an elastic volume data storing unit 118 configured to store the elastic image frame data output from the elastic scan converter 117 as the elastic volume data, and a specified cross-section elastic image constructing unit 119 configured to construct an arbitrary elastic image from the elastic volume data stored in the elastic volume data storing unit 118.

The RF signal frame data selecting unit 113 stores the plural pieces of RF signal frame data from the phasing and adding unit 107, and selects a pair of, i.e. two pieces of RF signal frame data from the stored RF signal frame data group. For example, the RF signal frame data selecting unit 113, at the same time as sequentially storing the RF signal frame data generated by the phasing adding unit 107 in time series, i.e. on the basis of the frame rate of the image and selecting the stored RF signal frame data (N) as first data, selects one piece of RF signal frame data (X) from among the temporally stored RF signal frame data group (N-1, N-2, N-3, ..., N-M) in the past. Here, N, M and X are index numbers given to the RF signal frame data, and are the positive integers.

Then the displacement measuring unit 114 executes one-dimensional or two-dimensional correlation process from the selected one pair of data pieces, i.e. RF signal frame data (N) and RF signal frame data (X), and obtains one-dimensional or two-dimensional displacement distribution in relation to the displacement or the moving vector, i.e. the direction and the size of the displacement in the biological tissue corresponding to the respective points on the tomographic image. Here, the block matching method is used for detecting moving vectors. The block matching method divides an image into blocks formed by NXN pixels, focuses on the block in a region of interest, searches the most approximated block to the focused block from the previous frame, and executes a process to determine the sample value by predictive encoding, i.e. the difference by referring to the searched block.

The elasticity information calculating unit 115 calculates the elasticity values of the strain or the elasticity modulus of the biological tissue corresponding to the respective points on the tomographic image from the moving vector output from the displacement measuring unit 114 and the pressure value output from a pressure measuring unit 121, and generates the signals of the elastic image, i.e. the elastic image frame data on the basis of the elasticity values.

At this time, the strain data is calculated by performing spatial differentiation on the moving distance, for example the displacement of the biological tissue. Also, the elasticity modulus data is calculated by dividing the change of pressure by the change of strain. For example, the displacement measured by the displacement measuring unit 114 is set as L(X) and the pressure measured by the pressure measuring unit 121 is set as P(X), the strain ΔS(X) can be calculated by performing the spatial differentiation on L(X) using the equation: ΔS(X)= ΔL(X)/ΔX. Also, the Young's modulus Ym(X) of the elasticity modulus data can be calculated by the equation: Ym=(ΔP(X))/ΔS(X). Since the elasticity modulus of the biological tissue corresponding to the respective points on the tomographic image can be obtained from this Young's modulus Ym, the 2-dimensional elastic image data can be consecutively obtained. The Young's modulus is the proportion of the simple tensile stress added to a subject with respect to the distortion generated in parallel to the tension.

The elastic image constructing unit 116 is configured including a frame memory and an image processing section, for storing the elastic image frame data output from the elasticity information calculating unit 115 in the frame memory in time series and executing image processing on the stored elastic image frame data. The elastic scan converter 117 executes coordinate conversion on the elastic image frame data from the elastic image constructing unit 116 to match the data to the display of the image display unit 109.

The elastic volume data storing unit 118 executes the 3-dimensional conversion on the plural pieces of elastic image frame data on the basis of the transmitting/receiving direction (*θ*,*φ*) equivalent to the acquisition position of the elastic image, generates and stores the elastic volume data. The elastic volume data in the elastic volume data storing unit 118 is read out to the specified cross-section elastic image constructing unit 119, and an elastic image with respect to the arbitrarily specified cross-section is constructed.

The ultrasonic diagnostic apparatus 101 comprises a synthesis processing unit 120 configured to perform processing for displaying a tomographic image, an elastic image, and a synthetic image in which a tomographic image and an elastic image are synthesized, and the image display unit 109 configured to display the images output from the synthesis processing unit 120.

Further, the ultrasonic diagnostic apparatus 101 comprises an operation unit 125 configured to arbitrarily specify an elastic image to be displayed on the image display unit 109 and a cross-section of the tomographic image, and a specified cross-section control unit 124 configured to control the specified cross-section tomographic image constructing unit 112 and the specified cross-section elastic image constructing unit 119 to display a tomographic image and an elastic image of the specified cross-section using the information of the specified cross-section which is specified by the operation unit 125. The operation unit 125 comprises devices such as a keyboard and a trackball.

The ultrasonic diagnostic apparatus 101 disclosed in the first embodiment comprises at least:
an ultrasonic probe 103 provided with transducers that transmits/receives ultrasonic waves;
a transmission unit 104 configured to transmit ultrasonic waves to the object 102 via the ultrasonic probe 103;
a reception unit 105 configured to receive the reflected echo signals from the object 102;
a volume data storing unit 111 and 118 configured to store the tomographic volume data and the elastic volume data created by processing the reflected echo signals;
an operation unit 125 configured to arbitrarily specify a cross-section;
a specified cross-section tomographic image constructing unit 112 configured to cut out and construct a tomographic image of the specified cross-section from the tomographic volume data stored in the volume data storing unit 111 and 118;
a specified cross-section elastic image constructing unit 119 configured to cut out and construct an elastic image of the cross-section from the elastic volume data stored in the volume data storing unit 111 and 118; and
an image display unit 109 configured to juxtapose and display the tomographic image and the elastic image constructed by the specified cross-section tomographic image constructing unit and the specified cross-section elastic image constructing unit.

The ultrasonic probe 103 may also be configured to execute ultrasonic scanning by a pressure control section 122 and a motor control section 123 for collecting the volume data. For example, the ultrasonic probe 103 can control the pressure to be applied vertically to the object 103 by the motor control unit 123. Also, the ultrasonic probe 103 can 3-dimensionally collect the RF signal frame data by moving the rectangle or fan-shaped plurality of transducers in the scan direction to collect the volume data. The motor control section 123 can also control to stop the movement of the plurality of transducers in the scan direction at the time of controlling the pressure.

The tomographic volume data storing unit 111 is also capable of acquiring information on the pressed position from the pressure control section 122, selecting and storing the tomographic image frame data of the same pressed position at all scanned positions in the minor-axis direction of the tomographic volume data, at the time of storing one volume of the tomographic image frame data output from the black and white scan converter 110. In the same manner, the elastic volume data storing unit 118 is also capable of acquiring the information on the pressed position from the pressure control section 122, selecting and storing the elastic image frame data of the same pressed position at all scanned positions in the minor-axis direction of the elastic volume data, at the time of storing one volume of the elastic image frame data output from the elastic scan converter 117.

Here, the process for displaying a specified cross-section will be described. An examiner arbitrarily specifies a cross-section of a tomographic image and an elastic image to be displayed on the image display unit 109 using the operation unit 125. In concrete terms, the examiner rotates the trackball of the operation unit 125, selects the position of a specified cross-section in the tomographic image and the elastic image to be displayed on the display unit 109, and specifies a cross-section by a determination key of the keyboard.

A specified cross-section control unit 124 outputs the control signal in accordance with the 3-dimensional positional information with respect to the specified cross-section tomographic image constructing unit 112 and the specified cross-section elastic image constructing unit 119 using the 3-dimensional positional information of the specified cross-section specified by the operation unit 125. The 3-dimensional positional information includes the information on the 3-dimensional position and the direction of the specified cross-section. In the case that plural specified cross-sections are specified, the control signal has the information on the number of specified cross-sections to be cut out.

The specified cross-section tomographic image constructing unit 112 inputs the tomographic volume data stored in the tomographic volume data storing unit 111 and the control signal from the specified cross-section control unit 124, and associates the spatial 3-dimensional position and the direction of the specified cross-section with the tomographic volume data. Then the specified cross-section tomographic image constructing unit 112 cuts out the tomographic image which is associated with the specified cross-section from the tomographic volume data, constructs a tomographic image in the specified cross-section and outputs the image to the synthesis processing unit 120.

The specified cross-section elastic image constructing unit 119 inputs the elastic volume data stored in the elastic volume data storing unit 118 and the control signal from the specified cross-section control unit 124, and associates the spatial 3-dimensional position and the direction of the specified cross-section with the elastic volume data. Then the specified cross-section elastic image constructing unit 119 cuts out the elastic image which is associated with the cross-section from the elastic volume data, constructs an elastic image in the specified cross-section and outputs the image to the synthesis processing unit 120.

Since the tomographic image constructed by the specified cross-section tomgraphic image constructing unit 112 and the elastic image constructed by the specified cross-section elastic image constructing unit 119 are cut out while being associated with each other by the 3-dimensoinal position and the direction in the same specified cross-section output from the specified cross-section control unit 124, the tomographic image and the elastic image are of the same specified cross sections.

The synthesis processing unit 120 executes the output processing of the tomographic images, elastic images and synthetic images in which a tomographic image and an elastic image are synthesized constructed by the specified cross-section tomographic image constructing unit 112 and the specified cross-section elastic image constructing unit 119. Here, the processing of synthetic images will be described. The synthesis processing unit 120 matches the position and the direction of a tomographic image with those of an elastic image on the basis of the 3-dimensional position and the direction in the specified cross-section. Then the synthesis processing unit 120 performs addition of the tomographic image and the elastic image at a predetermined rate of synthesis, and creates a synthetic image by executing hue conversion (for example, RGB conversion). For example, an elastic image is made translucent to be added at the time of synthesizing a tomographic image and an elastic image, so that a hard region in the elastic image and a low luminance region in the tomographic image can be compared.

Also, the synthesis processing unit 120 displays any of a tomographic image, elastic image and synthetic image in a specified cross-section, or selects plural images from among the tomgraphic image, elastic image and synthetic image for juxtaposing and displaying the selected images respectively. Then the image display unit 109 displays the images output from the synthesis processing unit 120.

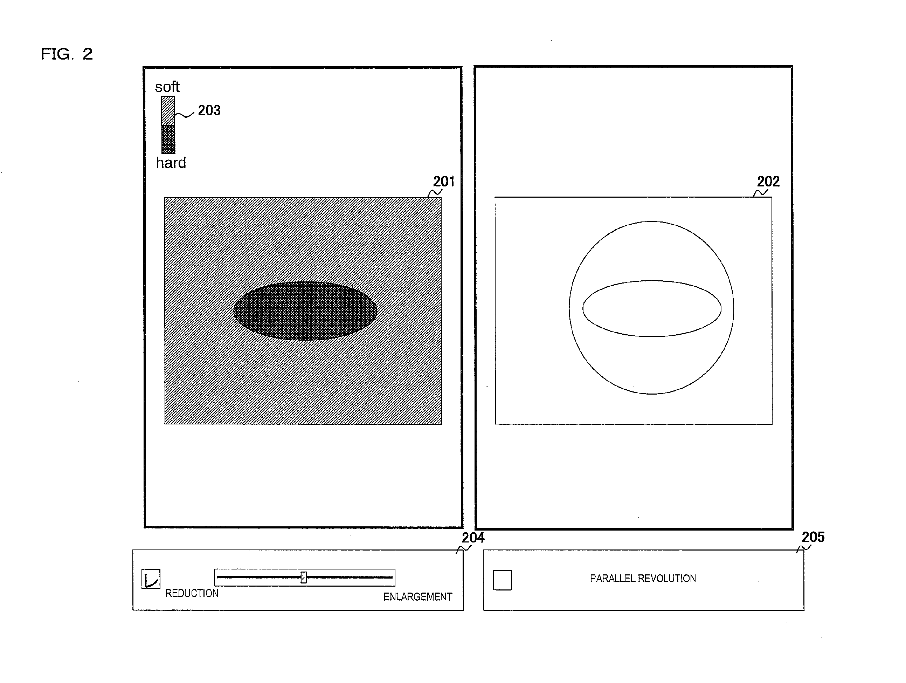

Here, a display pattern of a specified cross-section in the image display unit 109 will be described referring to Fig. 2. The image display unit 109 displays an elastic image 201 on the left side, and juxtaposes a tomographic image 202 of the same cross-section as the elastic image 201 on the right side. While the elastic image 201 is displayed on the left side of the image display unit 109 in the present embodiment, a synthetic image in which the elastic image 201 and the tomographic image 202 are synthesized may instead be displayed. Hereinafter, an elastic image or a synthetic image will be referred to as an elastic image for simple description. Also, the elastic image 201 is displayed along with a scale bar 203 for showing hardness.

The image display unit 109 is capable of synchronizing the elastic image 201 and the tomographic image 202 for enlarging, reducing or rotating the images. The image display unit 109 displays a scaling control panel 204 configured to synchronize the elastic image 201 and the tomographic image 202 for enlarging or reducing the images, and a parallel revolution control panel 205 configured to synchronize and rotate the elastic image 201 and the tomographic image 202. To the scaling control panel 204 and the parallel revolution control panel 205, a check box is provided to be checked when the panel is selected. In the case of synchronizing the elastic image 201 and the tomographic image 202 for scaling, the examiner checks the checkbox of the scaling control panel 204 using the operation unit 205, and moves the bar which is displayed in the scaling control panel 204 to the right or left. Scale of enlargement or reduction is set in accordance with the position of the bar.

In the case of changing the scale of enlargement or reduction by moving the bar displayed in the scaling control panel 204 to the right or left, the image display unit 109 executes the process for displaying the elastic image 201 and the tomographic image 202 with the same scale factor (scale of enlargement or reduction) respectively, and displays the elastic image 201 and the tomographic image 202. In other words, the scaled elastic image 201 and the tomographic image 202 are of the same cross-section displayed with the same scale factor, and the tissue of the object 102 to be displayed by the elastic image 201 and the tomographic image 202 are the same size.

Also, in the case of synchronizing and rotating the elastic image 201 and the tomographic image 201, the examiner checks the check box of the parallel revolution control panel 205 using the operation unit 205 and rotates the trackball of the operation unit 205. The rotation angle is set in accordance with the rotation of the trackball. When the elastic image 201 and the tomographic image 202 are rotated, the image display unit 109 executes image processing to rotate the images by setting the center of the respective screens as the central axis so that the elastic image 201 and the tomographic image 202 are displayed with the same rotation angles (same directions), and performs rotational display of the elastic image 201 and the tomographic image 202. In other words, the rotated elastic image 201 and the tomographic image 202 are of the same cross-section and have the same rotation angle, and the tissue of the object 102 to be displayed by the elastic image 201 and the tomographic image 202 are displayed in the same direction. In addition, the operation unit 205 is capable of arbitrarily setting the position of the central axes.

In accordance with the present embodiment, by juxtaposing and displaying the elastic image 201 having the elasticity information and the tomographic image 202 in the same cross-section as that of the elastic image 201 on the image display unit 109, the examiner can obtain the hardness information from the elastic image 201 and the information on the tissue structure from the tomographic image 202. Therefore, the examiner can make a diagnosis effectively by obtaining the biological information in relation to the object 102 without switching the screens.

While the elastic image 201 and the tomographic image 202 are displayed in parallel on the image display unit 109 in the present embodiment, the images can also be juxtaposed and displayed longitudinally. Also, the image display unit 109 can perform hue modulation on the elastic image 201 independently.

### Embodiment 2

The second embodiment will be described referring to Figs. 1 ~ 4. The difference from the first embodiment is that the image display unit 109, when an elastic image (or a synthetic image) displayed on the image display unit 109 is specified by the operation unit 125, displays the tomographic image of the same cross-section as that of the specified elastic image in parallel with the elastic image.

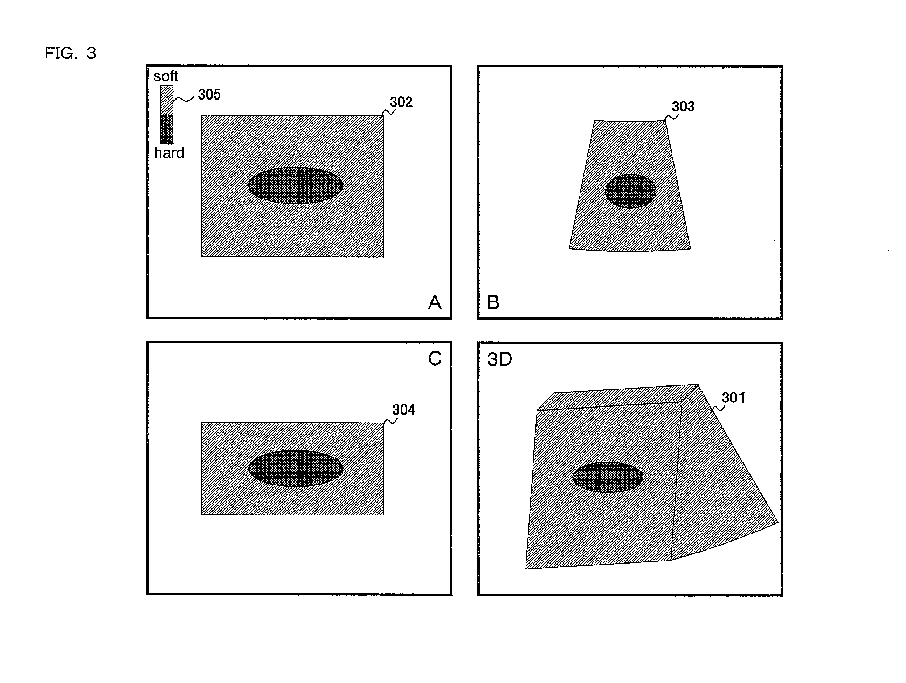

As shown in the MPR display pattern in Fig. 3, the image display unit 109 displays a rendering image 301 created on the basis of the tomographic volume data and the elastic volume data, and elastic images 302 ~ 304 in orthogonal three cross-sections of the tomographic volume data and the elastic volume data. The elastic images 302 ~ 304 are displayed with a scale bar 305 that shows hardness.

When the synthesis processing unit 120 executes rendering by associating the 3-dimensional position and the direction in the tomographic volume data stored in the tomographic volume data storing unit 111 with the elastic volume data stored in the elastic volume data storing unit 118, the image display unit 109 can display the rendering image 301. The elastic images 302 ~ 304 in the orthogonal three cross-sections are the elastic images cut out in the three cross-sections that are orthogonal to each other in the space of the tomographic volume data and the elastic volume data. The elastic image 302 ~ 304 in the orthogonal three cross-sections are constructed using the specified cross-section tomographic image constructing unit 112 and the specified cross-section elastic image constructing unit 119 as described in the first embodiment.

Here, the examiner specifies the cross-sections of the orthogonal three cross-sections for juxtaposing and displaying the elastic image and the tomographic image using the operation unit 125. In concrete terms, the examiner specifies any of cross-sections A ~ C corresponding to the elastic images 302 ~ 304 in the orthogonal three cross-sections using the operation unit 125.

The present embodiment will be described assuming that cross-section A is specified from among the orthogonal three cross-sections. The specified cross-section control unit 124 outputs the control signal having the 3-dimensional positional information to the specified cross-section tomographic image constructing unit 112 and the specified cross-section elastic image constructing unit 119 using the specified cross-section information in the cross-section A which is specified by the operation unit 125. The specified cross-section tomographic image constructing unit 112 cuts out the tomographic image which is associated with the specified cross-section of the cross-section A from the tomographic volume data, constructs the tomographic image in the specified cross-section in the cross-section A, and outputs the constructed image to the synthesis processing unit 120. The specified cross-section elastic image constructing unit 119 cuts out the elastic image which is associated with the specified cross section of the cross-section A, constructs an elastic image in the specified cross-section of the cross-section A and outputs the constructed image to the synthesis processing unit 120.

The synthesis processing unit 120 outputs the elastic image (or a synthetic image) and the tomographic image in the specified cross-section of the cross-section A that are constructed by the specified cross-section tomographic image constructing unit 112 and the specified cross-section elastic image constructing unit 119. Accordingly, as shown in Fig. 2, the image display unit 109 can juxtapose and display the elastic image 201 and the tomographic image 202 in any of the orthogonal three cross-sections.

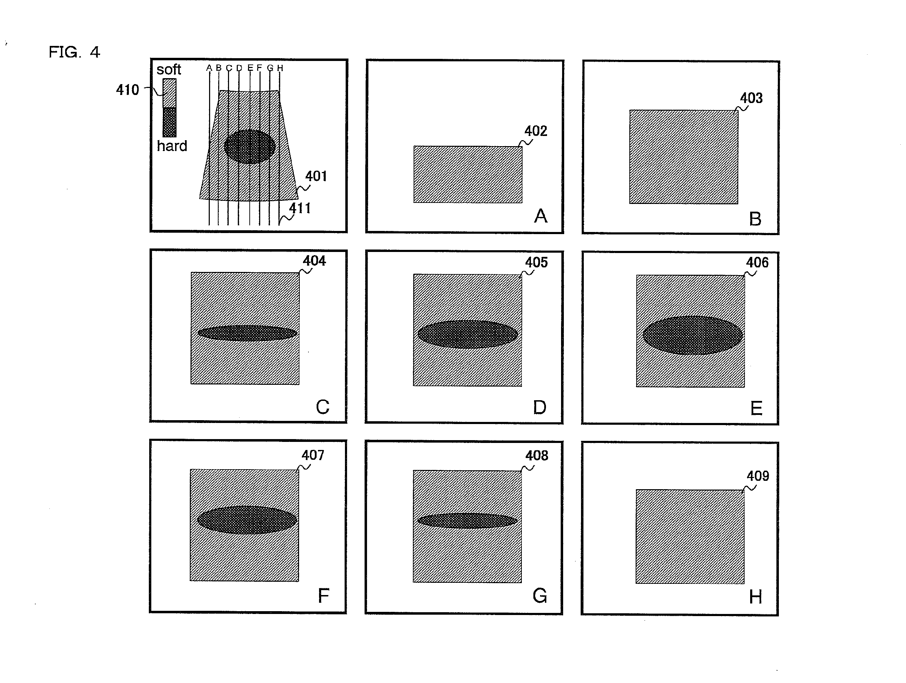

Also, as shown in the display pattern of parallel multi-sections of Fig. 4, the image display unit 109 can display an elastic image 401 in a predetermined specified cross-section and elastic images 402 ~ 409 that are multi-sections cut out in parallel from the elastic image 401. The elastic image 401 is displayed with a scale bar 410 that shows hardness.

The elastic images 402 ~ 409 are orthogonal to the elastic image 401, and are parallel to each other. Lines A ~ H indicated on the elastic image 401 correspond to the cross-sections A ~ H in the elastic images 402 ~ 409.

Here, the examiner specifies a cross-section in the parallel multi-sections which is for juxtaposing and displaying the elastic image and the tomographic image using the operation unit 125. In concrete terms, the examiner specifies one of the cross-sections A ~ H corresponding to the elastic images 402 ~ 409 in the parallel multi-sections as the specified cross-section using the operation unit 125.

The present embodiment will be described assuming that the cross-section D is specified from among the parallel multi-sections. The specified cross-section control unit 124 outputs the control signal having the 3-dimensoinal positional information to the specified cross-section tomographic image constructing unit 112 and the specified cross-section elastic image constructing unit 119 using the specified cross-section information in the cross-section D which is specified by the operation unit 125. The specified cross-section tomographic image constructing unit 112 cuts out the tomographic image which is associated with the specified cross-section of the cross-section D from the tomographic volume data, constructs the tomographic image in the specified cross-section of the cross-section D, and outputs the constructed image to the synthesis processing unit 120. The specified cross-section elastic image constructing unit 119 cuts out the elastic image which is associated with the specified cross-section of the cross-section D from the elastic volume data, constructs the elastic image in the specified cross-section of the cross-section D, and outputs the constructed image to the synthesis processing unit 120.

The synthesis processing unit 120 outputs the elastic image (or a synthetic image) in which a tomographic image and an elastic image are synthesized and the tomographic image in the specified cross-section of the cross-section D constructed by the specified cross-section tomographic image constructing unit 112 and the specified cross-section elastic image constructing unit 119. Thus as shown in Fig. 2, the image display unit 109 can juxtapose and display the elastic image 201 and the tomographic image 202 in any specified cross-section in the parallel multi-sections.

As mentioned above, the present embodiment is capable of, when an elastic image is specified by the operation unit 125 from among plural elastic images (or synthetic images) displayed on the display unit 109, juxtaposing and displaying the tomographic image of the same cross-section as that of the specified elastic image.

Therefore, the examiner is capable of, in the condition that a plurality of elastic images are displayed, making a diagnosis effectively by specifying a desired cross-section to be used for diagnosis, juxtaposing and displaying the elastic image and the tomographic image of the specified cross-section.

### Embodiment 3

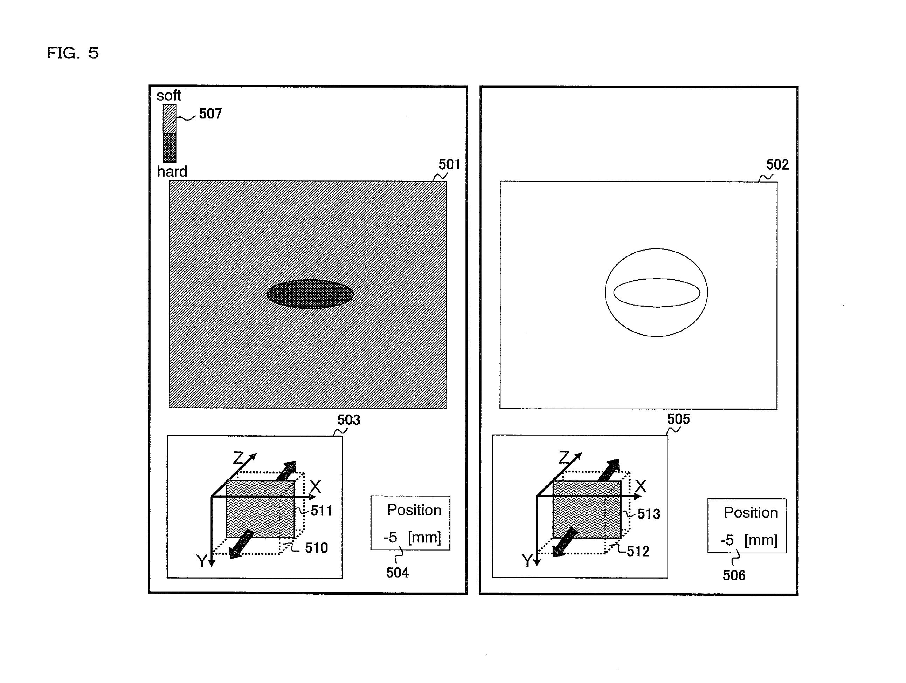

The third embodiment will be described referring to Figs. 1 and 5. The difference of the present embodiment from the first and second embodiments is that the image display unit 109 can display the positional information of the cross-section which is specified by the operation unit 125.

In the same method as in the first and second embodiments, an elastic image 501 (or a synthetic image), a tomographic image 502 of the same cross-section as that of the elastic image 501, and a scale bar 507 are displayed on the display unit 109.

Further, the positional information of the specified cross-section is also displayed on the image display unit 109. The image display unit 109 displays an orientation image 503 to be displayed so that the 3-dimensional position and the direction of the cross-section of the elastic image 501 can be visually identified and an orientation image 505 to be displayed so that the 3-dimensional position and the direction of the cross-section of the tomographic image 502 can be visually identified.

The orientation image 503 is formed by a rendering image 510 created on the basis of the tomographic volume data and the elastic volume data and a slice plane 511 which indicates the position of the specified cross-section of the elastic image 501. Also, the X-axis, Y-axis and Z-axis are displayed on the rendering image 510. In this manner, the examiner can recognize that the slice plane 511 corresponding to the specified cross-section is set on the X-Y plane.

The orientation image 505 is formed by a rendering image 512 created on the basis of the tomographic volume data and a slice plane 513 which indicates the position of the specified cross-section of the tomographic image 502. The X-axis, Y-axis and Z-axis are displayed on the rendering image 512. In this manner, the examiner can recognize that the slice plane 513 corresponding to the specified cross-section is set on the X-Y plane. The rendering image 510 and the rendering image 512 can also be replaced by a body mark which indicates the imaging area of the object 102 to be displayed.

Also, the image display unit 109 can display the positional information of the specified cross-section as numeric values 504 and 506. The numeric values 504 and 506 can be displayed, for example as -5mm, by setting the position of the Z-axis in the slice planes 511 and 513 as numeric values.

In this manner, the examiner can identify the positional information of the specified cross-section. While the pattern that the slice planes 511 and 513 corresponding to the specified cross-section is set on the X-Y plane is described in the present embodiment, the cross-section can also be set on the Y-Z plane, Z-X plane or other planes for displaying the positional information of the specified cross-section in the same manner as the above-described method.

### Embodiment 4

The fourth embodiment will be described referring to Figs. 1, 5 and 6. The difference of the present embodiment from the first ~ third embodiments, in the case of resetting a specified cross-section by the operation unit 125, is that the tomographic image and the elastic image (or a synthetic image) in the reset specified cross-section can be juxtaposed and displayed on the image display unit 109.

First, as shown in Fig. 5, the examiner synchronizes and moves the slice plane 511 and the slice plane 513 in the depth direction (Z-axis direction: arrow direction) using the operation unit 125, and resets the cross-section. The specified cross-section control unit 124 outputs the control signal in accordance with the 3-dimensional positional information to the specified cross-section tomographic image constructing unit 112 and the specified cross-section elastic image constructing unit 119 using the 3-dimensional positional information of the specified section which is reset by the operation unit 125.

Then as shown in the first embodiment, the specified cross-section tomographic image constructing unit 112 and the specified cross-section elastic image constructing unit 119 cut out the tomographic image and the elastic image that are associated with the reset cross-section on the basis of the control signal, construct the tomographic image and the elastic image in the specified cross-section, and outputs the constructed images to the synthetic processing unit 120. The synthetic processing unit 120 performs output processing of the tomographic image and the elastic image constructed by the specified cross-section tomographic image constructing unit 112 and the specified cross-section elastic image constructing unit 119. The image display unit 109 displays the tomographic image and the elastic image on the same cross-section that are associated and cut out by the 3-dimensional position and the direction in the reset specified cross-section.

Therefore, by changing the position of the specified cross-section of the tomographic image and the elastic image, the examiner can observe the in-vivo structure and the changing state of hardness of the tissue in the object 102.

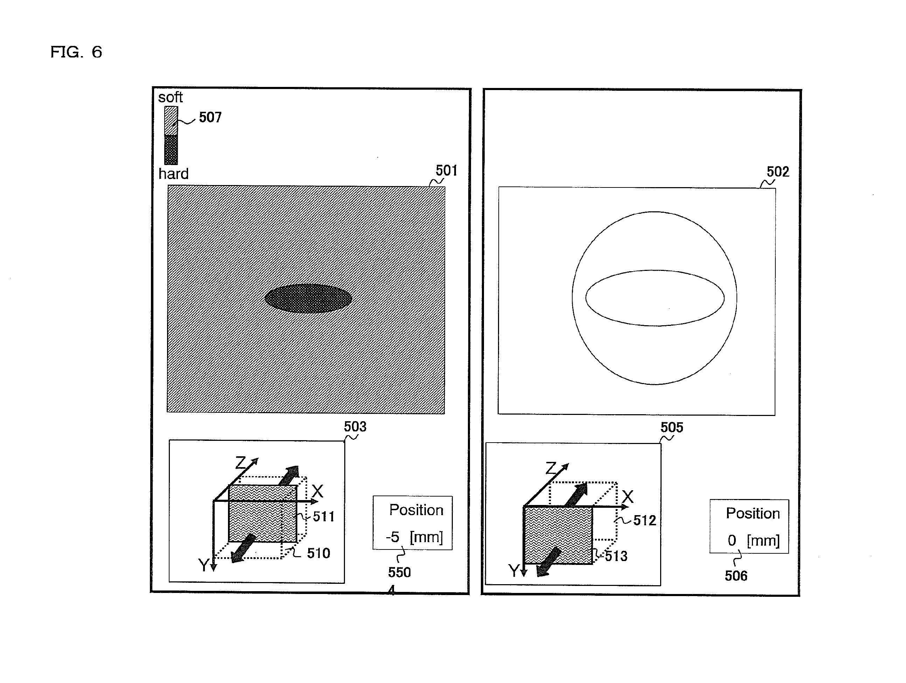

As shown in Fig. 6, the present embodiment also resets one of the specified cross-section of the tomographic image or the specified cross-section of the elastic image independently, and displays the tomographic image or the elastic image in the reset specified cross-section on the image display unit 109.

First, as shown in Fig. 6, the examiner moves only the slice plane 513 in the depth direction (Z-axis direction: arrow direction) using the operation unit 125, and resets the specified cross-section. The specified cross-section control unit 124 outputs the control signal in accordance with the 3-dimensional positional information to the specified cross-section tomographic image constructing unit 112 using the 3-dimensional positional information of the specified cross-section which is reset by the operation unit 125.

Then as indicated in the first embodiment, the specified cross-section tomographic image constructing unit 112 cuts out the tomographic image which is associated with the reset specified cross-section on the basis of the control signal, constructs a tomographic image in the specified cross-section, and outputs the constructed image to the synthesis processing unit 120. The synthesis processing unit 120 performs output processing of the tomographic image constructed by the specified cross-section tomographic image constructing unit 112. The image display unit 109 displays the tomographic image which is associated with and cut out by the 3-dimensional position and the direction in the reset cross-section. Since only the cross-section of the slice plane 513 is reset, the displayed tomographic image and the elastic image are of the different cross-sections.

In this manner, the examiner can observe the changing state of the in-vivo structure or the hardness of tissue in the object 102 by changing the position in the specified cross-section of the tomographic image or the elastic image.

### Embodiment 5

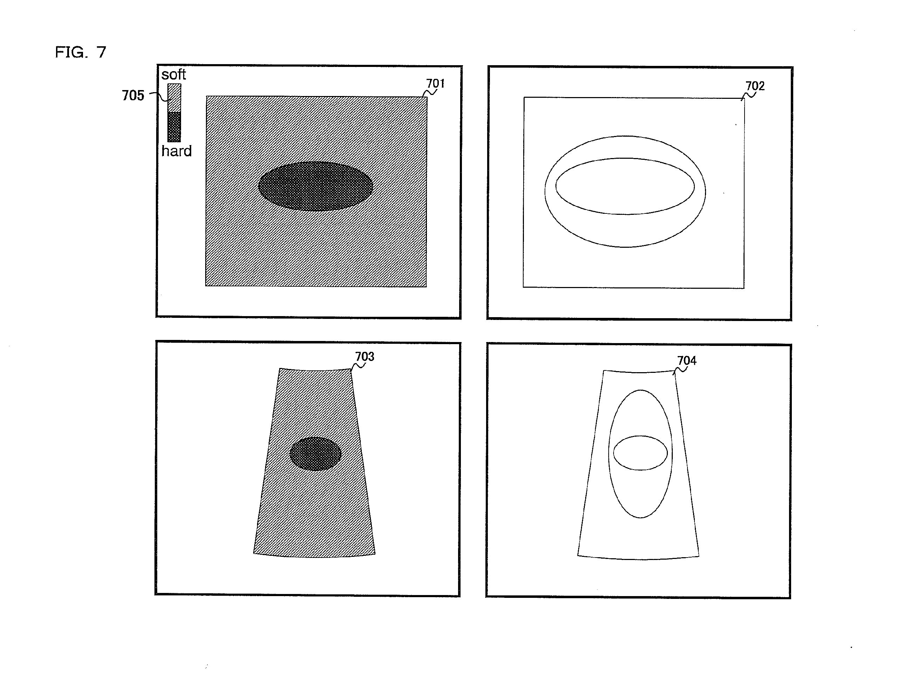

The fifth embodiment will be described referring to Figs. 1 and 7. The difference of the present embodiment from the first ~ fourth embodiments is, when plural specified cross-sections are set by the operation unit 125, that the tomographic images and the elastic images in the plural cross-sections can be juxtaposed and displayed on the display unit 109.

The examiner rotates the trackball of the operation unit 125, and selects the positions of the plural cross-sections of the tomographic images and the elastic images (or synthetic images) to be displayed on the display unit 109.

The specified cross-section control unit 124 outputs the control signal in accordance with the plural pieces of 3-diemnsional positional information to the specified cross-section tomographic image constructing unit 112 and the specified cross-section elastic image contrasting unit 119 using the plural pieces of 3-dimensional positional information set by the operation unit 125. Since plural cross-sections are specified, the control signal has the information on the number of specified cross-sections to be cut out. It is assumed in the present embodiment that two cross-sections are specified.

Then as shown in the first embodiment, the specified cross-section tomographic image constructing unit 112 and the specified cross-section elastic image constructing unit 119 cut out the tomographic image and the elastic image associated with the set plural specified cross-sections (two cross-sections) on the basis of the control signal, constructs the tomographic images and the elastic images in the plural cross-sections, and outputs the constructed images to the synthesis processing unit 120. The synthetic processing unit 120 performs output processing of the tomographic images and the elastic images constructed by the specified cross-section tomographic image constructing unit 112 and the specified cross-section elastic image constructing unit 119. The image display unit 109 displays the tomographic images and the elastic images in the plural cross-sections (two cross-sections) by the 3-dimensional position and the direction in the set plural cross-sections (two cross-sections). For example, as shown in Fig. 7, the image display unit 109 displays a tomographic image 702 and an elastic image 701, and a tomgoraphic image 703 and an elastic image 704 in the plural specified cross-sections (two specified cross-sections), with a scale bar 705.

In this manner, the examiner can observe the changing state of the in-vivo structure and the hardness of tissue in the object 102 by obtaining the information on the hardness of tissue from the elastic images 701 and 703 in the plural cross-sections as well as obtaining the information on the tissue structure from the tomographic images 702 and 704.

### Description of Reference Numerals

- 101:: ultrasonic diagnostic apparatus
- 102:: object
- 103:: ultrasonic probe
- 104:: transmission unit
- 105:: reception unit
- 106:: transmission/reception control unit
- 107:: phasing and adding unit
- 108:: tomographic image constructing unit
- 109:: image display unit
- 110:: black and white scan converter
- 111:: tomographic volume data storing unit
- 112:: specified cross-section tomographic image constructing unit
- 113:: RF frame data selecting unit
- 114:: displacement measuring unit
- 115:: elasticity information calculating unit
- 116:: elastic image constructing unit
- 117:: elastic scan converter
- 118:: elastic volume data storing unit
- 119:: specified cross-section elastic image constructing unit
- 120:: synthetic processing unit
- 121:: pressure measuring unit
- 122:: pressure control unit
- 123:: motor control unit
- 124:: specified cross-section control unit
- 125:: operation unit

## Claims

1. An ultrasonic diagnostic apparatus comprising:
an ultrasonic probe provided with transducers that transmit/receive ultrasonic waves;
a transmission unit configured to transmit ultrasonic waves to an object via the ultrasonic probe;
a reception unit configured to receive the reflected echo signals from the object;
a volume data storing unit configured to store tomographic volume data and elastic volume data created by processing the reflected echo signals;
an operation unit configured to arbitrarily specify a cross-section;
a specified cross-section tomographic image constructing unit configured to cut out and construct a tomographic image of the specified cross-section which is specified by the operation unit from the tomographic volume data stored in the volume data storing unit;
a specified cross-section elastic image constructing unit configured to cut out and construct an elastic image of the specified cross-section which is specified by the operation unit from the elastic volume data stored in the volume data storing unit; and
a display unit configured to juxtapose and display the tomographic image and the elastic image that are constructed by the specified cross-section tomographic image constructing unit and the specified cross-section elastic image constructing unit.

2. The ultrasonic diagnostic apparatus according to claim 1, wherein the tomographic image and the elastic image that are cut out in the specified cross-section are of the same cross-section.

3. The ultrasonic diagnostic apparatus according to claim 1, further comprising a synthesis processing unit configured to create a synthetic image by synthesizing the tomographic image and the elastic image, wherein the display unit displays the tomographic image and the synthetic image in parallel.

4. The ultrasonic diagnostic apparatus according to claim 1, further comprising a specified cross-section control unit configured to output a control signal in accordance with the 3-dimensional positional information to the specified cross-section tomographic image constructing unit and the specified cross-section elastic image constructing unit using the 3-dimensional positional information of the specified cross-section which is specified by the operation unit.

5. The ultrasonic diagnostic apparatus according to claim 1, wherein the 3-dimensional positional information includes the 3-dimensional position and the direction of the specified cross-section.

6. The ultrasonic diagnostic apparatus according to claim 1, wherein the specified cross-section tomographic image constructing unit associates the spatial 3-dimensinoal position and the direction of the specified cross-section with the tomographic volume data stored in the volume data storing unit, cuts out the tomographic image which is associated with the specified cross-section from the tomographic volume data, and constructs the tomographic image in the specified cross-section.

7. The ultrasonic diagnostic apparatus according to claim 1, wherein the specified cross-section elastic image constructing unit associates the special 3-dimensional position and the direction of the specified cross-section with the elastic volume data stored in the volume data storing unit, cuts out the elastic image which is associated with the specified cross-section from the elastic volume data, and constructs the elastic image in the specified cross-section.

8. The ultrasonic diagnostic apparatus according to claim 1, wherein the display unit displays the elastic image and the tomographic image in synchronization with the same scale of enlargement or reduction.

9. The ultrasonic diagnostic apparatus according to claim 1, wherein the display unit displays the elastic image and the tomographic image in synchronization with the same rotation angle.

10. The ultrasonic diagnostic apparatus according to claim 1, wherein the display unit, when the elastic image or the synthetic image is specified by the operation unit, displays in parallel the tomographic image in the same cross-section as that of the specified elastic image or the synthetic image.

11. The ultrasonic diagnostic apparatus according to claim 1, wherein the display unit displays the positional information of a specified cross-section which is specified by the operation unit.

12. The ultrasonic diagnostic apparatus according to claim 1, wherein the display unit, when the specified cross-section is reset by the operation unit, displays in parallel the tomographic image and the elastic image in the reset cross-section.

13. The ultrasonic diagnostic apparatus according to claim 1, wherein the display unit, when plural cross-sections are set by the operation unit, displays in parallel tomographic images and elastic images of the plural cross-sections.

14. An ultrasonic image display method including steps of:
transmitting ultrasonic waves to an object via an ultrasonic probe;
receiving the reflected echo signals from the object;
storing tomographic volume data and elastic volume data created by processing the reflected echo signals;
arbitrarily specifying a cross-section;
cutting out and constructing a tomographic image of the cross-section specified from the tomographic volume data;
cutting out and constructing an elastic image of the cross-section specified from the elastic volume data; and
displaying the constructed tomographic image and the elastic image in parallel to each other.
